# EUROPEAN PATENT SPECIFICATION

(11) **EP 2 436 433 B1**
(45) Date of publication and mention of the grant of the patent: **17.04.2013**
(21) Application number: 10185458.6
(22) Date of filing: 01.10.2010
(51) Int. Cl.: B01D 46/00, B60H 3/06

(54) **Odour preventing air filter assembly for a vehicle**
Geruchsverhindernde Luftfilteranordnung für ein Fahrzeug
Assemblage de filtre à air prévenant les odeurs pour véhicule

(43) Date of publication of application: 04.04.2012
(73) Proprietor: Volvo Car Corporation, 40 531 Göteborg (SE)
(72) Inventor: Lindroth, Patrik, 42257, Hisings Backa (SE); Libander, Patrik, 429 44, Särö (SE)

(56) References cited:
- EP-A1- 0 715 878
- CN-Y- 2 918 131
- FR-A1- 2 921 867
- US-A1- 2004 141 875

## Description

### TECHNICAL FIELD

The invention relates to an anti odour filter assembly for preventing growth of microbes on an air filter in accordance with the preamble of claim 1.

### BACKGROUND OF THE INVENTION

Vehicles in general are equipped with a climate unit for providing a pleasant and comfortable environment within a passenger compartment of the vehicle. However, air passing through the climate unit may sometimes comprise an odour. The odour normally commences when the vehicle has been used for a period of time. The odour may be apprehended as fairly unpleasant for persons within the passenger compartment. It has been found that the odour may be derived from e.g. bacteria, fungus or other microbes which have adhered against an air filter in e.g. the climate unit. It has further been found that accumulated water in the air filter also may be a cause of unpleasant odour within the vehicle. Environments which are wet and dark are found to be favorable for growth and propagation of microbes.

WO 2006/026818 A1 discloses a system arranged for preventing odour in the air within a vehicle. The odour in the air is controlled and prevented by a process reducing the quantity of particles in the air. In accordance with WO 2006/026818 A1, the particles are charged by a process of triboelectrification. The charged particles are attracted towards and against baffles arranged in the system. Thereafter, the particles adhered against the baffles are flushed away by water applied against the baffles.

CN 2 918 131 Y discloses a filter assembly according to the preamble of claim 1.

A drawback in prior art technology is that a process or apparatus arranged for reducing or preventing odour in the air within a vehicle passenger compartment may require many different components. The large numbers of components in such processes are dependent on each others functionalities. Hence, if one component fails to function it may have an impact on the functionality of the subsequent components within the process. Further, to produce and assemble many different components may negatively impact cost and production time.

### SUMMARY OF THE INVENTION

An object of the invention is to provide an anti odour filter assembly for preventing or reducing odour from microbes having adhered against an air filter in a vehicle air conditioning system.

These and other objects may be achieved by an anti odour filter assembly in accordance with claim 1.

As such, the present invention relates to an anti odour filter assembly for preventing growth of microbes on an air filter for a vehicle. The air filter is arranged for percolation of air flowing towards a passenger compartment of said vehicle. The anti odour filter assembly comprises an air filter and a microbe killing arrangement. In accordance with the invention, the microbe killing arrangement comprises means for emitting energy towards a surface of the filter. The means are arranged to be selectively activated for a period of time by an electronic control unit for killing microbes on said surface.

An advantage of the present invention is that the microbes having adhered on the surface of the air filter will die when energy is emitted towards the surface. The air percolating through the air filter will thus not comprise odour from the microbes. Dead microbes, compared to living microbes, leave no or a very small amount of odour.

Another advantage of the present invention is that the terminated microbes may remain on the air filter until regular service of the vehicle is performed. The air filter may thus be replaced with a new or cleaned air filter at the service.

Another advantage of the present invention is that the anti odour assembly requires a small amount of components compared to prior art assemblies. This makes the anti odour assembly cost and time efficient to produce and assemble.

The anti odour filter assembly may be activated selectively for a period of time. More particularly, the means for emitting energy towards the surface may be selectively activated for a period of time. The means for emitting energy can be activated at regular intervals. This has the advantage that the anti odour assembly do not have to be continuously active. Energy may thus be saved as electricity is required only when the assembly is active. Further, when the vehicle is not in active use, the means for emitting energy may be arranged to be activated at intervals. This could e.g. arise when the vehicle is parked over night, or for a longer period of time. During such periods, the vehicle may e.g. be plugged into an electrical grid. The means for emitting energy can then be activated at intervals without risking that the vehicle runs out of battery. The batteries of the vehicle can be charged simultaneously, upon being plugged in to the electrical grid.

The regular intervals for emitting energy may be determined by a sensor. The sensor may be arranged with the air filter. The sensor may communicate an activation signal to a processor when the quantity of active microbes has reached a level where risk for unpleasant odour arises in the air percolating through the air filter. The processor then communicates to the means for emitting energy towards the surface of the air filter.

According to the present invention, the means for emitting energy is arranged to emit an electrical field on or against the surface of the filter. Further, the electrical field is generated by a conductive element. The conductive element is electrified upon activation of the conductive element. The electrical field is arranged to kill the microbes on the surface of the air filter. The electrical field may consist of a generated electrical impulse from the conductive element. The purpose of the electrical impulse is to kill the microbes on the surface.

Preferably, the conductive element is in contact with the surface of the filter. When the conductive element generates the electrical impulse, the surface of the filter is electrified. Hence, the electrical impulse thus kills the microbes on the surface. Preferably, the conductive element comprises at least one conductive wire. The conductive wire may be arranged in a pattern forming a net on the surface of the air filter. This has the effect that the conductive element can cover a large area of the surface of the air filter.

According to the present invention, the electrical field is arranged to apply an electrical charge to the microbes, different from an internal charge of the microbes, thus killing the microbes. The microbes may have similar charge characteristics as particles. Hence, each microbe may have an internal charge. The electrical field with the electrical charge is arranged to differ from the internal charge of the microbes. When the microbes are arranged in the electrical field, the electrical charge and the internal charge will thus counteract each other. The counteracting charges have as a result that the microbes die.

Preferably, the filter comprises titanium dioxide. Titanium dioxide may function as a catalyst on the surface of the air filter when exposed to UV-light. The catalyst improves the effect of killing the microbes on the surface.

### BRIEF DESCRIPTION OF THE DRAWINGS

The present invention will hereinafter be explained in greater detail by means of nonlimiting examples and with reference to the appended drawings in which:
- Fig. 1: illustrates an air filter of a vehicle arranged in a flow path being exposed to UVlight,
- Fig. 2: illustrates an air filter of a vehicle arranged in a flow path being exposed to a hot airstream,
- Fig. 3: illustrates an air filter of a vehicle arranged in a flow path being exposed to an electrical field from a conductive element against a surface of the air filter,
- Fig. 4: illustrates an air filter of a vehicle arranged in a flow path being exposed to an electrical field in accordance with Fig. 3 where the conductive element is arranged as a net against the surface of the air filter.

It should be noted that the appended drawings are not necessarily drawn to scale and that the dimensions of some features of the present invention may have been exaggerated for the sake of clarity.

### DETAILED DESCRIPTION OF PREFERRED EMBODIMENTS

The invention will, in the following, be exemplified by embodiments. It is to be understood, however, that the embodiments are included in order to explain principles of the invention and not to limit the scope of the invention defined by the appended claims.

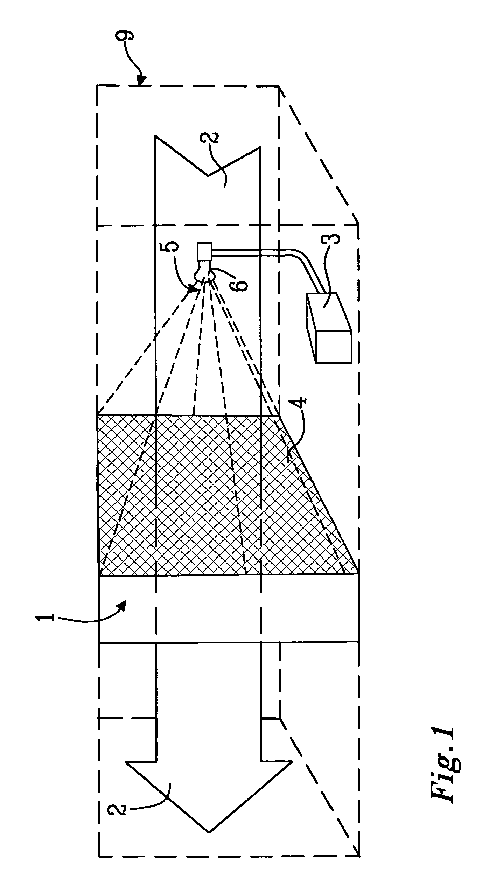

Fig 1 - 4 illustrates an anti odour filter assembly to be arranged within a vehicle. The anti odour filter assembly comprises an air filter 1 and a microbe killing arrangement. The air filter 1 is arranged in an air 2 flow path 9 towards a passenger compartment of the vehicle. The microbe killing arrangement comprises an electronic control unit 3 and means for emitting energy towards a surface 4 of the air filter 1. Microbes, which have adhered on the surface 4 of the air filter 1, may cause a bad and unpleasant odour in the air 2 which percolates there through. Means for emitting energy towards the surface 4 of the air filter 1 may be selectively activated for a period of time by the electronic control unit 3. The energy emitted against the surface 4 may be arranged for killing a majority of the microbes thereon.

In accordance with one comparative example, Fig. 1 illustrates the air filter 1 being arranged in the air 2 flow path 9 towards the passenger compartment. Means for emitting energy in the form of UV-light 5 is arranged in the flow path 9 upstream the air filter 1. UV-light 5 is arranged to be emitted towards the surface 4. At least one UV-lamp 6 is used for emitting UV-light 5. The UV-lamp 6 is arranged to illuminate the surface 4 with UV-rays or UV-light. The electronic unit 3 may be arranged to control the level of UV-illumination of the UV-lamp 6.

In general, microbes are sensitive to light, especially to UV-light. Therefore, UV-light is found to be effective for killing microbes and preventing growth thereof.

The air filter 1 may comprise titanium dioxide. Titanium dioxide functions as a catalyst on the filter 1 when exposed to UV-light. An enhanced process of killing the microbes on the surface 4 of the air filter 1 may thus be achieved.

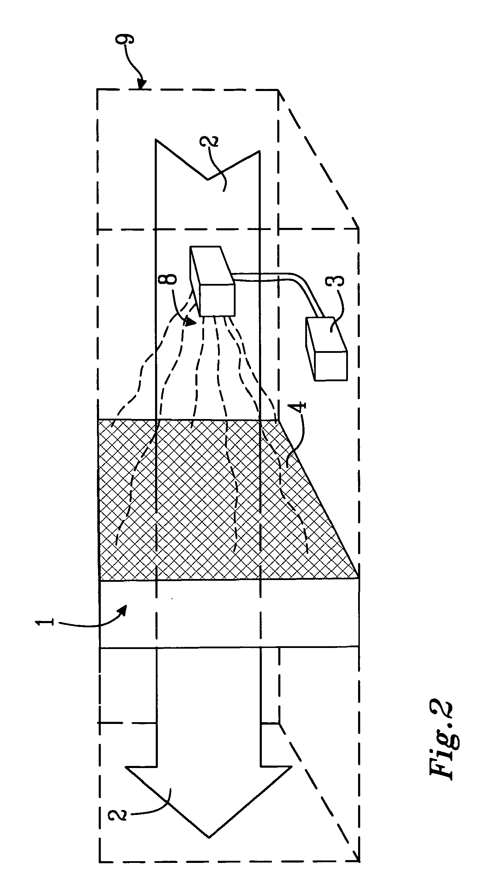

In accordance with another comparative example, Fig. 2 illustrates the air filter 1 being arranged in the air 2 flow path 9 towards the passenger compartment. Means for emitting energy in the form of a hot or warm airstream 8 is arranged in the flow path 9 upstream the air filter 1. The airstream 8 is directed towards the surface 4 of the air filter 1. The means for emitting energy rises the temperature of the airstream 8 to a temperature level high enough for killing a majority of the microbes adhered on the surface 4. The temperature of the airstream 8 may exceed 60 degrees Celsius. A temperature level about 60 degrees Celsius or more is preferable. At such a level, a sufficient amount of microbes will be killed on the air filter 1 for avoiding or reducing odour in the percolating air. In accordance with the invention, the air which is warmed up and thereafter provided as an airstream 8 towards the surface 4 may comprise air which originates either from the passenger compartment or from outside the vehicle. After the warm air has percolated through the air filter 1 it continues towards the passenger compartment.

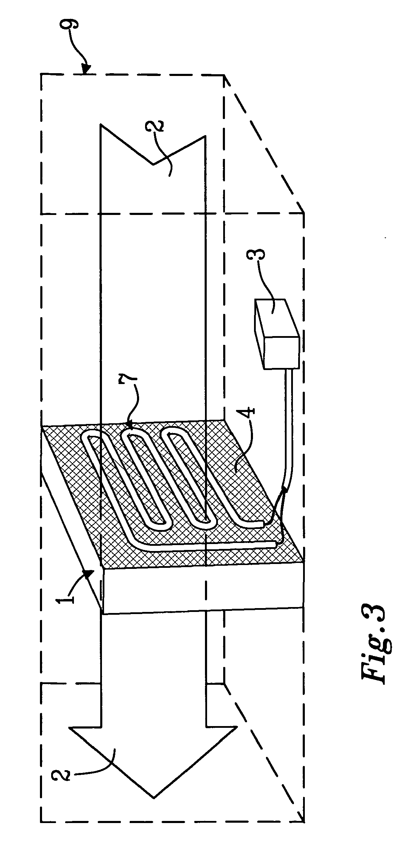

In accordance with an embodiment of the invention, Fig. 3 illustrates the air filter 1 being arranged in the air 2 flow path 9 towards the passenger compartment. Means for emitting energy is arranged on the surface 4 of the air filter 1 facing upstream in the flow path 9. In accordance with Fig. 3, the means for emitting energy is arranged to emit an electrical field on or against the surface 4. A conductive element 7 is arranged to provide the electrical field. The conductive element 7 may be in contact with the surface 4. The conductive element 7 comprises at least one conductive wire. The wire may be arranged on the surface 4 in a pattern forming e.g. a net. Other patterns of the wire may also be applicable. Example of other patterns may e.g. be serpentine pattern or Z pattern. Further, the wire may extend in a back and forward pattern over the surface 4. A gap may be provided between two adjacent parts of the wire when extending in the back and forward pattern over the surface 4. A current through the wire may provide an electrical field extending perpendicular from the wire. The electrical field may thus be emitted towards and against the surface 4. The adjacent parts of the wire has to be arranged close enough to each other in order to enable killing of microbes between the adjacent wire parts.

A control unit 3 communicates with the wire, thus provides the current to the wire.

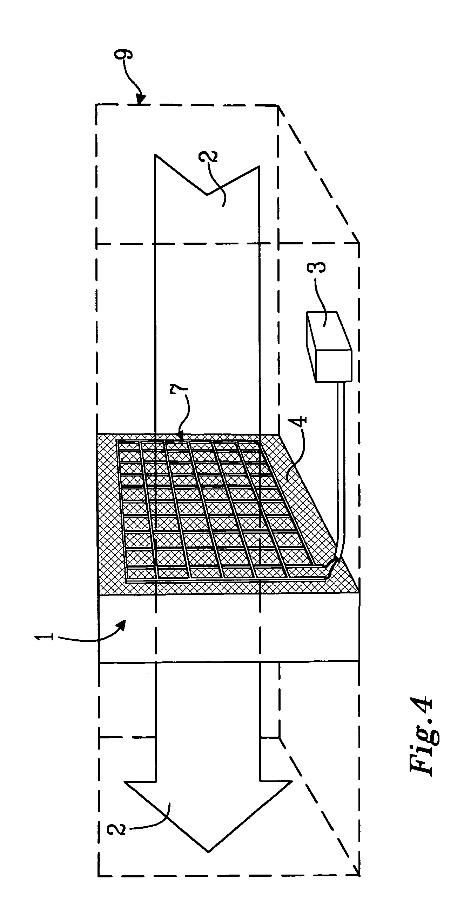

Fig, 4 illustrates an alternative embodiment in accordance with the invention, as illustrated in Fig. 3. In Fig. 4 the conductive element 7 is arranged with the wire in the shape of a net applied against the surface 4. However, as mentioned above other solutions for applying the wire against the surface 4 may be possible. Another further alternative solution may be to e.g. print the conductive element on the surface 4 of the air filter 1.

The conductive element may provide an electrical field for killing the microbes in at least two ways. The conductive element may be arranged to provide an electrical shock against the surface 4, for killing the microbes there on. Further, the conductive element may be arranged to provide an electrical charge within the conductive element. Microbes in general may be seen as equivalents to particles. Microbes comprise an internal charge, thus allowing them to behave as charged particles. The electrical charge applied differs from the internal charge of the microbes. The electrical charge applied and the internal charge of the microbes will counteract each other when the microbes are exposed to the electrical charge. The counteracting charges thus results in that the microbes are killed.

The dead microbes may remain on the air filter 1 until the air filter 1 is changed. The air filter 1 may be changed at regular intervals. The regular intervals may e.g. occur during service of the vehicle. The air filter 1 being changed may be washed and cleaned or replaced with a new air filter.

While there have been shown and described and pointed out fundamental novel features of the invention as applied to a preferred embodiment thereof, it will be understood that various omissions and substitutions and changes in the form and details of the devices illustrated, and in their operation, may be made by those skilled in the art. It is the intention, therefore, to be limited only as indicated by the scope of the claims appended hereto.

## Claims

1. An anti odour filter assembly for preventing growth of microbes on an air filter (1) for a vehicle, wherein said air filter (1) is arranged for percolation of air (2) flowing towards a passenger compartment of said vehicle, wherein said anti odour filter assembly comprises said air filter (1) as well as a microbe killing arrangement, and wherein the microbe killing arrangement comprises means for emitting energy towards a surface (4) of the air filter (1), **characterized in that** the means for emitting energy is arranged to emit an electrical field on or against the surface (4) of the air filter (1) and that this electrical field is arranged to apply an electrical charge to the microbes different from an internal charge of the microbes, thus killing the microbes.

2. An anti odour filter assembly according to claim 1, **characterized in that** the electrical field is generated by a conductive element (7).

3. An anti odour filter assembly according to claim 2, **characterized in that** the conductive element (7) is in contact with the surface (4) of the air filter (1).

4. An anti odour filter assembly according to any one of claims 2 or 3, **characterized in that** the conductive element (7) comprises at least one conductive wire.

5. An anti odour filter assembly according to claim 4, **characterized in that** the conductive wire is arranged to form a net.

6. An anti odour filter assembly according to any one of the preceding claims, **characterized in that** the means for emitting energy is arranged to be selectively activated for a period of time by an electronic control unit (3).

7. An anti odour filter assembly according to claim 6, **characterized in that** the electronic control unit (3) comprises a sensor which is arranged to communicate an activation signal to a processor for activation of the means for emitting energy when the quantity of active microbes on the filter has reached a level where risk for unpleasant odours arises in the air flowing through the air filter.

8. An anti odour filter assembly according to any one of the preceding claims, **characterized in that** the filter (1) comprises titanium dioxide.

## Patentansprüche

1. Geruchsschutz-Filterbaugruppe zum Verhindern des Wachstums von Mikroben auf einem Luftfilter (1) für ein Fahrzeug, wobei der Luftfilter (1) zur Filtration von Luft (2), die zu einem Fahrgastraum des Fahrzeugs hin strömt, angeordnet ist, wobei die Geruchsschutz-Filterbaugruppe den Luftfilter (1) sowie eine Mikroben abtötende Anordnung umfasst und wobei die Mikroben abtötende Anordnung Mittel zum Freisetzen von Energie zu einer Oberfläche (4) des Luftfilters (1) hin umfasst, **dadurch gekennzeichnet, dass** die Mittel zum Freisetzen von Energie dafür angeordnet sind, ein elektrisches Feld an oder gegen die Oberfläche (4) des Luftfilters (1) freizusetzen, und dass dieses elektrische Feld dafür angeordnet ist, eine elektrische Ladung an die Mikroben anzulegen, die sich von einer inneren Ladung der Mikroben unterscheidet, was folglich die Mikroben abtötet.

2. Geruchsschutz-Filterbaugruppe nach Anspruch 1, **dadurch gekennzeichnet, dass** das elektrische Feld durch ein leitfähiges Element (7) erzeugt wird.

3. Geruchsschutz-Filterbaugruppe nach Anspruch 2, **dadurch gekennzeichnet, dass** sich das leitfähige Element (7) in Kontakt mit der Oberfläche (4) des Luftfilters (1) befindet.

4. Geruchsschutz-Filterbaugruppe nach einem der Ansprüche 2 oder 3, **dadurch gekennzeichnet, dass** das leitfähige Element (7) wenigstens einen leitfähigen Draht umfasst.

5. Geruchsschutz-Filterbaugruppe nach Anspruch 4, **dadurch gekennzeichnet, dass** der leitfähige Draht so angeordnet ist, dass er ein Netz bildet.

6. Geruchsschutz-Filterbaugruppe nach einem der vorhergehenden Ansprüche, **dadurch gekennzeichnet, dass** die Mittel zum Freisetzen von Energie dafür angeordnet sind, durch eine elektronische Steuereinheit (3) selektiv für einen Zeitraum aktiviert zu werden.

7. Geruchsschutz-Filterbaugruppe nach Anspruch 6, **dadurch gekennzeichnet, dass** die elektronische Steuereinheit (3) einen Sensor umfasst, der dafür angeordnet ist, ein Aktivierungssignal an einen Prozessor weiterzuleiten, zur Aktivierung der Mittel zum Freisetzen von Energie, wenn die Menge von aktiven Mikroben auf dem Filter ein Niveau erreicht hat, bei dem eine Gefahr von unangenehmen Gerüchen in der durch den Luftfilter strömenden Luft entsteht.

8. Geruchsschutz-Filterbaugruppe nach einem der vorhergehenden Ansprüche, **dadurch gekennzeichnet, dass** der Filter (1) Titandioxid umfasst.

## Revendications

1. Ensemble filtre anti-odeur servant à empêcher la croissance des microbes sur un filtre à air (1) pour un véhicule, lequel filtre à air (1) est conçu pour assurer la percolation d'air (2) s'écoulant en direction d'un habitacle dudit véhicule, lequel ensemble filtre anti-odeur comprend ledit filtre à air (1) ainsi qu'un aménagement microbicide, et lequel aménagement microbicide comprend un moyen servant à émettre de l'énergie en direction d'une surface (4) du filtre à air (1), **caractérisé en ce que** le moyen servant à émettre de l'énergie est conçu pour émettre un champ électrique sur ou contre la surface (4) du filtre à air (1) et **en ce que** ce champ électrique est conçu pour appliquer aux microbes une charge électrique différente d'une charge interne des microbes de façon à les tuer.

2. Ensemble filtre anti-odeur selon la revendication 1, **caractérisé en ce que** le champ électrique est généré par un élément conducteur (7).

3. Ensemble filtre anti-odeur selon la revendication 2, **caractérisé en ce que** l'élément conducteur (7) est au contact de la surface (4) du filtre à air (1).

4. Ensemble filtre anti-odeur selon l'une quelconque des revendications 2 ou 3, **caractérisé en ce que** l'élément conducteur (7) comprend au moins un fil conducteur.

5. Ensemble filtre anti-odeur selon la revendication 4, **caractérisé en ce que** le fil conducteur est conçu pour former un treillis.

6. Ensemble filtre anti-odeur selon l'une quelconque des revendications précédentes, **caractérisé en ce que** le moyen servant à émettre de l'énergie est conçu pour être activé de façon sélective pendant un intervalle de temps par une unité de commande électronique (3).

7. Ensemble filtre anti-odeur selon la revendication 6, **caractérisé en ce que** l'unité de commande électronique (3) comprend un capteur qui est conçu pour transmettre un signal d'activation à un processeur pour activer le moyen servant à émettre de l'énergie si la quantité de microbes actifs présents sur le filtre atteint un niveau tel que des odeurs désagréables risquent de se former dans l'air s'écoulant à travers le filtre à air.

8. Ensemble filtre anti-odeur selon l'une quelconque des revendications précédentes, **caractérisé en ce que** le filtre (1) comprend du dioxyde de titane.
